# FASCICULE DE BREVET EUROPEEN

(11) **EP 1 257 596 B1**
(45) Date de publication et mention de la délivrance du brevet: **05.11.2003**
(21) Numéro de dépôt: 00990052.3
(22) Date de dépôt: 11.12.2000
(51) Int. Cl.: C08K 9/00, C08J 3/00, A61L 15/00

(54) **PROCEDE D'ADDITIVATION MINIMISANT LA SEGREGATION DES ADDITIFS ET LEUR MIGRATION**
VERFAHREN ZUR ZUSAMMENSETZUNG UNTER MINIMIERUNG DER ADDITIVTRENNUNG UND IHRER WANDERUNG
ADDITIVE MIXING METHOD REDUCING TO A MINIMUM ADDITIVE SEGREGATION AND MIGRATION

(30) Priorité: 13.12.1999 FR 9915671
(43) Date de publication de la demande: 20.11.2002
(73) Titulaire: Atofina, 92800 Puteaux (FR)
(72) Inventeur: GANCET, Christian, F-64140 Lons (FR); CHAMBRETTE, Jean-Paul, F-92400 Courbevoie (FR); GOURMAND, Myriam, F-75015 Paris (FR)
(74) Mandataire: Rieux, Michel
(86) Numéro de dépôt international: FR0003468
(87) Numéro de publication internationale: WO01044355

(56) Documents cités:
- WO-A-98/20915
- WO-A-99/41298
- US-A- 4 842 593

## Description

La présente invention se rapporte au domaine des polymères superabsorbants destinés à la réalisation d'articles sanitaires aptes à absorber et à retenir les liquides corporels, et plus particulièrement aux polymères superabsorbants renforcés contre les odeurs. Elle décrit en particulier un procédé d'incorporation empêchant la sédimentation des additifs.

Les polymères superabsorbants au sens de l'invention, désignés ci-après par SAP sont des polymères qui résultent de la polymérisation avec réticulation partielle de monomères éthyléniquement insaturés hydrosolubles, en particulier les acides acryliques et méthacryliques et leurs sels alcalins, qu'ils soient obtenus par un procédé de polymérisation en solution, en masse ou en suspension inverse. Ces polymères sont doués d'une très grande capacité d'absorption et de rétention de l'eau et des solutions aqueuses, et aujourd'hui largement répandus dans le commerce sous forme de poudres avec des granulométries restant comprises entre 100 et 800µm. La littérature en est très riche ; on pourra consulter par exemple EP-A-0312952 ou EP-A-0441507.

L'industrie des superabsorbants (SAP) est très souvent confrontée à un besoin d'ajout d'additif. En effet, pour répondre aux demandes du marché, il est souvent nécessaire d'ajouter d'autres propriétés à ces produits en plus de leurs performances d'absorption et de rétention. Par exemple, lorsque l'article absorbant en place est imprégné de liquides corporels, en particulier d'urine, il s'y dégage des odeurs puissantes et incommodantes. Parmi celles-ci dominent des odeurs ammoniacales dues à l'ammoniac provenant de l'hydrolyse de l'urée par les uréases des bactéries présentes sur la peau et dans le tube digestif.
Dans le but de supprimer ces odeurs il est nécessaire d'ajouter dans la composition un additif anti-microbien. Dans ce cas un tel composé est généralement qualifié d'agent ou d'additif anti-odeur

Pour ajouter un anti-odeur aux SAP, différentes techniques ont été utilisées. Ainsi WO 9820915 et EP 739635 décrivent des mélanges contenant respectivement des zéolites et du borax. Ces mélanges sont réalisés à l'état solide en mélange poudre-poudre.
US 4842593 décrit des changes contenant du SAP avec des agents tampons et un agent anti-microbien non-toxique, non-irritant et non volatil.

L'agent anti-microbien est préféré sous forme solide plutôt que sous forme liquide en solution à base d'alcool. Dans ce dernier cas, il n'est pas incorporé dans le SAP mais dans le voile (top sheet) au contact avec la peau.

Le procédé le plus utilisé pour ajouter un produit à un autre est le mélange à l'état solide dit incorporation. En effet, l'incorporation est un procédé efficace pour apporter de nouvelles fonctions à un produit industriel existant. De nombreux exemples peuvent être cités tels que les différents additifs (colorants, pigments, charges...) que l'on introduit dans les poudres de polymères comme le PVC, le PE, le PP et le polyamide avant extrusion.

Toutefois, ce type d'additivation conduit généralement à une ségrégation des composés, le composé le plus dense ayant tendance à sédimenter, et ce d'autant plus que la morphologie et la distribution de taille des 2 types de particules sont très différentes. Ce problème est d'autant plus crucial que les poudres de superabsorbants peuvent être stockées plusieurs mois et sont soumises à des conditions de transport pouvant favoriser la sédimentation (mer houleuse, route défoncée...).
EP 739635 et WO 9820915 proposent une solution basée sur l'adaptation de la granulométrie pour éviter la séparation et la sédimentation.
L'invention présentée ici entend résoudre ce phénomène de ségrégation dans le cas d'additif organique ayant une température de fusion < 200°C.

La solution proposée est basée sur un procédé permettant d'éviter les phénomènes de sédimentation et d'inhomogénéité dans les superabsorbants mélangés avec des composés organiques sous forme solide. Ce procédé est plus simple et moins coûteux qu'une incorporation dans le SAP par pulvérisation, imprégnation ou immersion avec une solution liquide aqueuse ou non car il n'y a pas d'étape de pulvérisation ni d'étape d'évaporation.

Le procédé de l'invention consiste, à chauffer le mélange SAP / additif, après homogénéisation à l'état solide, à une température comprise entre la température de fusion de l'additif et 200°C et de préférence supérieure d'au moins 5 °C à la température de fusion du produit. On a alors enrobage des particules de SAP par l'additif fondu, ce qui évite ensuite tout phénomène de ségrégation entre le SAP et l'additif et de migration de l'additif.

Selon l'invention l'additif anti-odeur est un anti-microbien tel que le TRICLOSAN décrit dans « the index of antimicrobials ; par Michael et Irene ASH ; édition GOWER - 1996 »

Le procédé de l'invention est tout à fait adapté à l'incorporation dans les SAP de l'IRGASAN DP300 qui est une formulation particulière du TRICLOSAN. En effet, dans le cas particulier de l'IRGASAN DP300, ce composé se présente sous la forme de bâtonnets de granulométrie moyenne 200µm, alors que les particules de SAP généralement utilisés en hygiène (type gel process ou suspension inverse) ont un diamètre compris entre 100 et 800µm (préférentiellement centré autour de 400-500µm) et des formes de cailloux (gel process) ou de billes agglomérées (suspension inverse). Ainsi, malgré une densité voisine de celle des particules de SAP, la ségrégation entre l'additif et la matrice est favorisée.

Ce procédé général est décrit plus particulièrement dans le cas de l'additivation des SAP par le TRICLOSAN, afin d'obtenir un SAP anti-odeur. Cet additif étant très efficace, la concentration optimale à introduire est comprise entre 0.01 et 0.5% (préférentiellement de l'ordre de 0.05%). Il est donc très important d'éliminer tout risque de ségrégation pour éviter des variations de dosage en TRICLOSAN dans l'application finale (changes bébé ou adulte et serviettes pour l'hygiène féminine). Il est également important de contrôler tout risque de migration des additifs vers la peau des bébés ou des adultes.

Selon une forme de l'invention la quantité nécessaire de l'agent anti-odeur est ajoutée directement dans le SAP, selon une autre forme on prépare tout d'abord un mélange maître SAP/additif ayant approximativement 5 % en poids d'additif, ensuite on dilue le mélange maître avec un SAP brut de façon à atteindre la teneur en additif nécessaire pour l'application.

Un autre objet de l'invention est un SAP contenant de 0,01 à 0,5 % en poids d' additif anti-bactérien et réalisé selon le procédé décrit précédemment.

Un autre objet de l'invention est l'utilisation des SAP dans les articles d'hygiène tels que les changes pour bébés ainsi que les articles d'hygiène eux-mêmes.

Pour réaliser un SAP renfermant 0.05% en poids de TRICLOSAN selon l'invention on peut procéder de la manière suivante :
1. On réalise tout d'abord un mélange maître à 5% en TRICLOSAN selon les étapes suivantes :
   a) homogénéisation du mélange SAP + TRICLOSAN dans un mélangeur.
   b) étuvage dynamique ou statique en fûts chauffants type mélangeur avec double enveloppe ou mélangeur suivi d'une capacité chauffante (l'homogénéisation initiale peut se faire dans ces fûts ou dans un autre mélangeur) ou de tout autre façon (par exemple à l'aide d'un brassage d'air chaud à une température supérieure à la température de fusion de l'additif désignée par Tf, ce qui permet le mélangeage et la fusion de l'additif) ou d'autres types d'appareils pouvant combiner ces deux actions
   ou statique (sous forme de sacs de mélange maître empilés sur une palette).
   La température d'étuvage doit être supérieure d'au moins 5°C à la température de fusion de l'additif organique. Elle est choisie en fonction de la quantité à traiter et du système utilisé et contrôlée grâce à des sondes placées au coeur du système. Dans le cas de l'IRGASAN DP300 (Tf = 57±1°C), il est préférable de chauffer le système SAP + additif à au moins 63°C, voire 65°C. Il faut toutefois que la température d'étuvage soit inférieure à la température de décomposition du produit. les performances de gonflement des SAP ne sont pas affectées par l'étuvage ( A 200°C, le SAP peut être étuvé environ 1 heure et beaucoup plus pour des températures inférieures).
   La durée d'étuvage est bien évidemment fonction de la différence de température entre la température d'étuvage et celle de fusion, ainsi que de la quantité de produit étuvé et du produit lui-même (par sa conductivité thermique). L'utilisation de mélange maître permet de diminuer la quantité de produit à étuver et par conséquent la durée d'étuvage.
   Un test rapide permet d'estimer la durée d'étuvage : une observation sous optique grossissante permet de confirmer la disparition des particules d'additif (bâtonnets dans le cas de l'IRGASAN DP300).
2. Refroidissement du système par retour à la température ambiante ou par refroidissement contrôlé soit
   - En statique
   - En continuant le mélangeage si l'étuvage a été réalisé en dynamique
   Après la fusion, l'additif organique reste un certain temps en surfusion, c'est-à-dire qu'il ne recristallise pas immédiatement quand la température devient inférieure à son point de fusion.
   Le mode de refroidissement, en statique ou en dynamique influence uniquement la « consistance » du produit après étuvage. En effet, un SAP fortement concentré en additif (cas du mélange maître) refroidit de façon statique (en palette) aura tendance à former des agglomérats (ces agglomérats sont facilement friables et peuvent facilement être désagglomérés par un mélangeage efficace ultérieurement). Il est toutefois préférable que le produit refroidisse sous agitation afin d'éviter ce phénomène d'agglomération observable avec un refroidissement statique.
3. Dilution dans un rapport 1 :100 du mélange maître dans du SAP brut.

Les exemples suivants illustrent l'invention sans en limiter la portée.

### EXEMPLES

### 1) Evaluation de la sédimentation avec des SAP additivés de 5% de TRICLOSAN avec et sans étuvage

### Description du test

Un tube en plexiglas de hauteur 42.5cm et de diamètre 3cm et fermé à ses 2 extrémités par des bouchons en caoutchouc est rempli de 150g de SAP additivé à 5% en TRICLOSAN. Ce tube (rempli au 2/3) est ensuite placé verticalement dans une tamiseuse de type RETSCH et est soumis à 10 minutes de vibrations (cycles de 10s de vibration et 3s de repos) d'amplitude 1.2mm afin de favoriser la sédimentation du mélange.

Environ 15 g de SAP sont ensuite récupéré par le haut et le bas du tube pour que la concentration en TRICLOSAN dans le SAP aux extrémités du tube puisse être déterminée.

### Conditions de préparation des échantillons

### Sans étuvage : mélange à sec pendant 2h au turbula de SAP HP200 + 5% triclosan

*Avec étuvage statique* : mélange à sec pendant 2h au turbula de SAP HP200 + 5% triclosan, suivi d'un étuvage à 75°C pendant 1h. L'échantillon est ensuite remis à température ambiante jusqu'à refroidissement complet et recristallisation du triclosan. Les agglomérats sont grossièrement éliminés par agitation avec une spatule puis l'échantillon est homogénéisé 2h au turbula.

*Avec étuvage dynamique* : mélange à sec pendant 2h au turbula de SAP HP200 + 5% triclosan. 400g de ce mélange sont introduits dans un réacteur en verre à double enveloppe de 1 litre muni d'un agitateur à pâles. La température de consigne du bain thermostaté est fixée à 80°C et la vitesse d'agitation à 350 tr/min. Au bout de 20 minutes de chauffe, le réacteur est refroidit. L'agitation est maintenu pendant 30 minutes pour laisser le temps au triclosan de recristalliser.

### Extraction du TRICLOSAN et dosage par UV

Le spectre UV du triclosan comporte 2 pics à lambda = 202.5 et 282 nm avec des coefficients d'extinction différents. Les méthodes A et B décrites ci-dessous diffèrent par la prise d'échantillon et le choix de la longueur d'onde. La méthode B sera préférée car la prise d'échantillon plus importante permet de limiter les erreurs dues à l'échantillonnage et, de plus, l'éthanol absolu utilisé comme solvant ne présente aucune absorbance à la longueur d'onde utilisée.

### Méthode A :

Pour des concentrations de l'ordre de 5% en TRICLOSAN, 1g de SAP additivé est ajouté dans 300g d'éthanol absolu et la solution est placée sous agitation à 500tr/mi pendant 2h puis soumise à 15min d'ultrasons. 5g du sumageant sont ensuite dilués dans 200g d'éthanol absolu afin d'obtenir une concentration finale mesurable par spectrophotométrie (absorbance comprise entre 0.5 et 1).
La concentration en TRICLOSAN dans des solutions d'éthanol absolu est déterminée par spectromètre UV. Une courbe d'étalonnage a ensuite été réalisée à la longueur d'onde lamda = 202.5 nm à partir de solutions témoins de concentrations comprises entre 1 et 8 ppm. L'introduction du surnageant dans les cellules de mesure se fait à l'aide d'une seringue et d'un filtre (pour éviter d'introduire des poussières). La référence utilisée pour les mesures de TRICLOSAN extrait de SAP est le surnageant obtenu selon le même protocole avec du SAP non additivé (temps d'extraction et dilutions identiques).

### Méthode B :

Pour des concentrations de l'ordre de 5% en TRICLOSAN, 10g de SAP additivé est ajouté dans 150g d'éthanol absolu et la solution est placée sous agitation à 500tr/min pendant 2h puis soumise à 15min d'ultrasons. 1g du surnageant est ensuite dilué dans 100g d'éthanol absolu afin d'obtenir une concentration finale mesurable par spectrophotométrie (absorbance comprise entre 0.5 et 1).
La concentration en TRICLOSAN dans des solutions d'éthanol absolu est déterminée par spectromètre UV. Une courbe d'étalonnage a ensuite été réalisée à la longueur d'onde lamda = 282 nm à partir de solutions témoins de concentrations comprises entre 10 et 60 ppm. L'introduction du surnageant dans les cellules de mesure se fait à l'aide d'une seringue et d'un filtre (pour éviter d'introduire des poussières). La référence utilisée pour les mesures de TRICLOSAN extrait de SAP est le surnageant obtenu selon le même protocole avec du SAP non additivé (temps d'extraction et dilutions identiques).

### Résultats

Les résultats sont la moyenne de deux extractions au minimum, chacune étant dosée au minimum 2 fois.

| | Concentration en triclosan dans le SAP (%) | |
|---|---|---|
| | Méthode A | Méthode B |
| HP200 + 5% triclosan Sans étuvage Fraction haute | 2.5 | 2.6 |
| HP200 + 5% triclosan Sans étuvage Fraction basse | 9.8 | 11.2 |
| HP200 + 5% triclosan Avec étuvage statique Fraction haute | 5.3 | - |
| HP200 + 5% triclosan Avec étuvage statique Fraction basse | 4.9 | - |
| HP200 + 5% triclosan Avec étuvage dynamique Fraction haute | 5.2 | 5.2 |
| HP200 + 5% triclosan Avec étuvage dynamique Fraction basse | 4.6 | 4.8 |

On peut noter qu'une très forte sédimentation de l'additif est observée lorsque le mélange SAP+ TRICLOSAN n'est pas étuvé. Au contraire, de fortes vibrations ne modifient pas la répartition de l'anti-bactérien dans le SAP lorsque le mélange a été étuvé.

### 2) Evaluation des performances anti-bactériennes et anti-odeur des SAP additivés de 0.05% de TRICLOSAN avec et sans étuvage.

### Préparation de l'urine

Le test peut être réalisé soit sur urine réelle, soit sur urine synthétique. Dans le cas de l'urine réelle, 4 jours avant le test, une collecte d'urine est réalisée avec indication de s'abstenir pour les personnes prenant des médicaments ou des antibiotiques. Pour l'urine synthétique, il suffit de la préparer le moment venu, selon la composition ci-après :

| Pour 1 I H₂O | | | |
|---|---|---|---|
| Urée | 25 g | K₂SO₄ | 4 g |
| NaCl | 9 g | (NH₄)₂SO₄ | 2,5 g |
| MgSO₄ | 0,6 g | Glucose | 5 g |
| Ca(OCOCH₃)₂ | 0,7 g | Extrait de levure | 5 g |

### TEST PANEL

### Préparation de l'inoculum

L'inoculum est préparé avec 20 ml d'urine réelle ou synthétique, 0,5 g d'urée et soit 2 g de fluff souillé (présentant déjà une odeur ammoniacale), soit la ou les souche(s) bactérienne(s) souhaitée(s). Le mélange est mis à incuber pour 2 jours, pendant lesquels l'urine collectée est conservée à 4°C.
Au moment du test, l'inoculum présente une odeur marquée, signe d'une croissance satisfaisante.
Dans le cas des souches isolées, il est procédé à une mesure de la concentration en bactéries*, afin de procéder à un ensemencement reproductible.
* exprimée en cfu/ml (colony forming units/ml)

### Préparation des échantillons

On prépare *n* x 8 boîtes hermétiques cylindriques en polyéthylène, *n* étant le nombre de produits à tester. Chaque série de 8 boîtes est repérée par un nombre aléatoire à trois chiffres. Dans chaque boîte, on dépose 0.5 g de cellulose (fluff), et 0.3 g de superabsorbant dispersé ou non dans la masse, additionné ou non du ou des produits anti-odeur à tester.
Sur chaque tampon, on verse 30 ml d'urine synthétique enrichie de la quantité souhaitée d'inoculum. Les boîtes sont refermées et mises à incuber une nuit à 37°C en étuve.

### Evaluation de l'odeur

Au moment du test, les boîtes sont sorties de l'étuve, et proposées de façon aléatoire aux personnes du jury qui doivent noter l'odeur entre 1 et 6. L'absence d'odeur de NH₃ est notée 1, et une odeur très forte est notée 6.
Les résultats sont rassemblés, et on calcule pour chaque produit testé la moyenne des notes obtenues.

### Comptage des bactéries

Après évaluation de l'odeur, il est procédé à un comptage des micro-organismes pour chaque type d'échantillon. Pour ce faire, on dilue les échantillons avec 70 ml d'eau stérile, et on procède au comptage à l'aide de plaquettes Millipore. Le résultat est exprimé en cfulml (colony forming units/ml).

### Exemple

Urine synthétique.
Inoculation à 10⁴ cfu/ml

| Echantillon | Note Panel Moyenne (1-6) | Bactéries, cfu/ml |
|---|---|---|
| HP200 témoin | 4.88 | TNTC* |
| HP200 + 0.01% Irgasan avec étuvage | 1.25 | 72-100 |
| HP200 + 0.01% Irgasan sans étuvage | 1.13 | 80-100 |
| HP200 + 0.05% Irgasan avec étuvage | 1.5 | 35-40 |
| HP200 + 0.05% Irgasan sans étuvage | 1.38 | 55-60 |
| HP200 + 0.1% Irgasan avec étuvage | 1.25 | 5-15 |
| HP200 + 0.1% Irgasan sans étuvage | 1.50 | 0-1 |

| | | |
|---|---|---|
| *Too numerous to count | | |

### TEST NH3

### Inoculation de l'urine synthétique

Un tube de bactéries Proteus mirabilis lyophilisées (Référence LMG 2954 - conservation à 4°**C** des bactéries lyophilisées) est introduit dans 333g d'urine synthétique (soit l'équivalent de 3 doses par litre).

### Préparation des échantillons

On prépare *n* x 2 fioles erlenmeyer de 100cm³ (rodage 19/26) comportant un raccord Torion (diamètre 8 sur rodage 19/26 et joint étanche en PTFE), *n* étant le nombre de produits à tester, dans lesquelles sont introduit 0.5g de fluff et 0.5g de SAP additivé de 0.05% de TRICLOSAN (étuvé ou non), dispersé ou non dans la masse de fluff. Sur chaque tampon, on verse 33g d'urine synthétique inoculée. Les fioles sont alors fermées par l'introduction d'un tube dosimètre prêt à la mesure (Tube GASTEC - passive dosi-tube NH3 - Référence PROLABO 02 436 112) puis placée en étuve à 40°C. La coloration du tube doseur permet de déterminer la concentration en NH₃ (en ppm) dans la fiole selon la formule suivante :

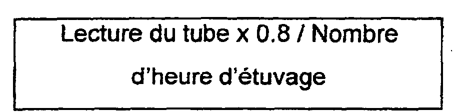

La valeur 0.8 du coefficient correspond à une température d'étuvage de 40°C.

### Résultats

| Durée (heure) | Dosage NH3 (ppm) | | |
|---|---|---|---|
| | Blanc (Fluff) | HP200 + 0.05% TRICLOSAN Avec étuvage | HP200 + 0.05% TRICLOSAN Sans étuvage |
| 0 | 0 | 0 | 0 |
| 1 | 0 | 0 | 0 |
| 2 | 5 | 0 | 0 |
| 3.5 | 6 | 0 | 0 |
| 4 | 7 | 0 | 0 |
| 5 | 16 | 0 | 0 |
| 6 | 60 | 0 | 0 |
| 7 | Hors échelle | 1 | 1 |
| 8 | Hors échelle | 1 | 1 |
| 9.5 | Hors échelle | 1 | 1 |
| 24 | Hors échelle | 1 | 1 |

La fabrication des systèmes SAP HP200 + 0.05% triclosan est réalisée à partir de mélange maître HP200 + 5% triclosan dilué 100 fois dans du SAP HP200.

*Système HP200 + 0.05% triclasan sans étuvage* : le mélange maître utilisé est celui réalisé sans étuvage (voir descriptif dans le paragraphe précédent). La dilution dans HP200 se fait par agitation 2h au turbula.

*Système HP200 + 0.05% triclosan avec étuvage* : le mélange maître utilisé est celui réalisé avec étuvage statique (voir descriptif dans le paragraphe précédent). La dilution dans HP200 se fait par agitation 2h au turbula.

On peut noter qu'il n'y a aucune différences significative des propriétés anti-bactériennes et anti-odeur du SAP lorsque le mélange maître est étuvé ou non (dans les conditions où le système non étuvé a été homogénéisé pour éviter toute sédimentation et par conséquent toute variation de concentration en TRICLOSAN)

## Revendications

1. Procédé d'enrobage de particules de polymères superabsorbants dit SAP par un additif anti-microbien consistant en :
a-le mélangeage et le malaxage d'une composition contenant de 90 à 99,99 % en poids de polymère et de 0.01 à 10 % en poids d'au moins un additif anti-microbien,
b- l'étuvage jusqu'à la fusion totale de l'additif à une température comprise entre la température de fusion de l'additif et 200°c.

2. Procédé selon la revendication 1 **caractérisé en ce que** ladite composition est constituée de 0,01 à 5% en poids d'additif

3. Procédé selon la revendication 1 ou 2 **caractérisé en ce que** la température d'étuvage est au moins de 5 degrés supérieure à la température de fusion de l'additif.

4. Procédé selon l'une des revendications précédentes **caractérisé en ce que** le mélangeage est réalisé à l'état solide.

5. Procédé selon l'une des revendications précédentes **caractérisé en ce que** l'étuvage est réalisé en statique

6. Procédé selon l'une des revendication 1 à 4 **caractérisé en ce que** l'étuvage est réalisé en dynamique

7. Procédé selon l'une des revendications précédentes **caractérisé en ce que** l'additif est le TRICLOSAN

8. Composition superabsorbantes tel qu'on peut l'avoir selon l'une quelconque des revendications précédentes

9. Utilisation des compositions superabsorbantes de la revendication 8 éventuellement diluées de façon à ramener la teneur en additif anti-microbien à une valeur comprise entre 0.01 et 0.5% en poids dans la réalisation d'articles sanitaires aptes à absorber et à retenir les liquides corporels.

10. Articles sanitaires tels que changes pour bébés, adultes et pour l'hygiène féminine contenant les SAP de la revendication 8.

## Patentansprüche

1. Verfahren zur Umhüllung von Partikeln aus superabsorbierenden Polymeren, die als SAP bezeichnet werden, mit einem antimikrobiellen Zusatzstoff, das die folgenden Schritte umfaßt:
a) Mischen und Kneten einer Zusammensetzung, die 90 bis 99,99 Gew.-% Polymer und 0,01 bis 10 Gew.-% mindestens eines antimikrobiellen Zusatzstoffs enthält,
b) Trocknen durch Erwärmen bis auf eine Temperatur, die im Bereich von der Schmelztemperatur des Zusatzstoffs bis zu einer Temperatur von 200 °C liegt, bis zum vollständigen Schmelzen des Zusatzstoffs.

2. Verfahren nach Anspruch 1, **dadurch gekennzeichnet, daß** die Zusammensetzung 0,01 bis 5 Gew.-% des Zusatzstoffs enthält.

3. Verfahren nach Anspruch 1 oder 2, **dadurch gekennzeichnet, daß** die Temperatur beim Trocknen durch Erwärmen mindestens 5 ° über der Schmelztemperatur des Zusatzstoffs liegt.

4. Verfahren nach einem der vorhergehenden Ansprüche, **dadurch gekennzeichnet, daß** das Vermischen im festen Zustand durchgeführt wird.

5. Verfahren nach einem der vorhergehenden Ansprüche, **dadurch gekennzeichnet, daß** das Trocknen durch Erwärmen unter statischen Bedingungen durchgeführt wird.

6. Verfahren nach einem der Ansprüche 1 bis 4, **dadurch gekennzeichnet, daß** das Trocknen durch Erwärmen unter dynamischen Bedingungen durchgeführt wird.

7. Verfahren nach einem der vorhergehenden Ansprüche, **dadurch gekennzeichnet, daß** es sich bei dem Zusatzstoff um Triclosan handelt.

8. Superabsorbierende Zusammensetzungen, die nach einem der vorhergehenden Ansprüche erhältlich sind.

9. Verwendung der superabsorbierenden Zusammensetzungen nach Anspruch 8, die gegebenenfalls verdünnt sind, um den Gehalt an antimikrobiellem Zusatzstoff auf einen Wert im Bereich von 0,01 bis 0,5 Gew.-% zu bringen, zur Herstellung von Hygieneartikeln oder Sanitärartikeln, die imstande sind, Körperflüssigkeiten zu absorbieren und zurückzuhalten.

10. Hygieneartikel oder Sanitärartikel, wie Windeln für Säuglinge, Erwachsene und Binden für die weibliche Hygiene, die die SAPs nach Anspruch 8 enthalten.

## Claims

1. Process for coating particles of superabsorbent polymers, known as SAPs, with an antimicrobial additive, which consists in:
a- mixing and blending a composition containing from 90% to 99.99% by weight of polymer and from 0.01% to 10% by weight of at least one antimicrobial additive,
b- incubating until the additive has totally melted at a temperature between the melting point of the additive and 200°C.

2. Process according to Claim 1, **characterized in that** the said composition consists of from 0.01% to 5% by weight of additive.

3. Process according to Claim 1 or 2, **characterized in that** the incubation temperature is at least 5° above the melting point of the additive.

4. Process according to one of the preceding claims, **characterized in that** the mixing is carried out in the solid state.

5. Process according to one of the preceding claims, **characterized in that** the incubation is carried out in static mode.

6. Process according to one of Claims 1 to 4, **characterized in that** the incubation is carried out in dynamic mode.

7. Process according to one of the preceding claims, **characterized in that** the additive is Triclosan.

8. Superabsorbent polymer composition as may be obtained according to any one of the preceding claims.

9. Use of the superabsorbent polymer compositions of Claim 8, optionally diluted so as to bring the content of antimicrobial additive to a value of between 0.01% and 0.5% by weight, to prepare sanitary articles capable of absorbing and retaining bodily fluids.

10. Sanitary article such as a diaper for babies and adults and for feminine hygiene, containing the SAPs of Claim 8.
